# DEMANDE DE BREVET EUROPEEN

(11) **EP 1 543 802 A2**
(43) Date de publication de la demande: **22.06.2005**
(21) Numéro de dépôt: 04356189.3
(22) Date de dépôt: 03.12.2004
(51) Int. Cl.: A61F 2/38

(54) **Prothèse de genou antéro-postéro-stabilisée à plateau tibial pivotant**

(30) Priorité: 15.12.2003 FR 0314672
(71) Demandeur: Biomet Merck France, 26903 Valence Cedex (FR); ORA, 69160 Tassin la Demi Lune (FR); Maestro, Michel, 06200 Nice (FR); Descamp, Loÿs, 06330 Nice (FR); Ovadia, Hervé, 06670 Colomars (FR); Puch, Jean-Marc, 06330 Roquefort les Pins (FR); Fabre, Thierry, 33200 Bordeaux (FR); Arnould, Hervé, 01000 Bourg en Bresse (FR)
(72) Inventeur: Augoyard, Marc, 69160 Tassin La Demi Lune (FR); Basso, Maurice, 83400 Hyeres (FR); Charret, Philippe, 69270 Fontaine sur Saone (FR); Debiesse, Jean-Louis, 38200 Vienne (FR); Dupre La Tour, Laurent, 42580 L'Etrat (FR); Eyraud, Guy, 38150 Ville Sous Anjou (FR); Fayard, Jean-Philippe, 42170 St Just St Rambert (FR); Hulin, Paul-Henri, 89000 Auxerre (FR); Lecuire, François, 83400 Hyeres (FR); Melere, Gilles, 74370 St. Martin de Bellevue (FR); Millon, Joseph, 73490 La Ravoire (FR); Passot, Jean-Paul, 42530 St Genest Lerpt (FR); Peyrot, Jacques, 69160 Tassin la Demi Lune (FR); Relave, Marc, 42160 Andrezieux Boutheon (FR); Maestro, Michel, 06200 Nice (FR); Descamp, Loÿs, 06330 Nice (FR); Ovadia, Hervé, 06670 Colomars (FR); Puch, Jean-Marc, 06330 Roquefort les Pins (FR); Fabre, Thierry, 33200 Bordeaux (FR); Arnould, Hervé, 01000 Bourg en Bresse (FR); Vernizeau, Michel, 26120 Upie (FR); de Witte, Gérard, 26300 Chateauneuf sur Isère (FR)
(74) Mandataire: Le Cacheux, Samuel L.R.

(57) **Abrégé**

La prothèse de genou selon l'invention est caractérisée en ce que :
■ la portée de l'élément prothétique fémoral (**1**) possède, d'une part, en section droite transversale par rapport à un plan sagittal, une forme concave, douce sans arête vive, et, d'autre part, en section droite selon le plan sagittal, une forme convexe,
■ et la rampe de glissement de l'insert tibial (**2**) est destinée à coopérer avec la partie (33) de l'élément fémoral (**1**) et possède, d'une part, en section droite transversale par rapport au plan sagittal, une forme convexe, douce sans arête vive, et, d'autre part, en section droite selon le plan sagittal, une forme concave, et se trouve destinée à coopérer avec la portée de l'élément (**1**) fémoral pour induire un mouvement de recul vers l'arrière, dit de « roll back » du fémur lors de la flexion de la prothèse.

## Description

La présente invention a pour objet les prothèses articulaires et elle concerne, plus particulièrement, le domaine des prothèses de genou.

Par prothèses du genou, il convient de retenir les systèmes articulaires artificiels visant à remplacer l'articulation naturelle constituée par la conformation épiphysaire basse du fémur, par la conformation épiphysaire complémentaire haute du tibia, voire par l'élément fémoro-patellaire.

La technique antérieure a formulé un grand nombre de propositions se rapportant au domaine technique ci-dessus.

Il a ainsi été proposé des prothèses totales, dites liées, en ce sens qu'elles font intervenir deux pièces complémentaires qui sont réunies par un système articulaire matériel, tel qu'au moins un axe, constituant le système de pivotement artificiel matérialisant l'articulation du genou, selon une direction perpendiculaire au plan sagittal ou antéro-postérieur.

Il a aussi été proposé des prothèses, dites libres, qui sont constituées, par opposition aux précédentes, à base de deux éléments, respectivement adaptables sur les épiphyses basse du fémur et haute du tibia, pour coopérer par glissement relatif en étant maintenus en contact de surface par l'intermédiaire, notamment, des ligaments latéraux interne et externe naturels, sans faire intervenir la présence d'un lien d'articulation matériel entre ces deux éléments.

Ainsi, certaines prothèses, dites liées ou libres, ont été conçues pour répondre à une exigence de restauration articulaire mettant en oeuvre une cinématique correspondant à un mouvement à deux degrés de liberté en rotation, au jeu près, à savoir un degré de liberté en rotation correspondant à un mouvement de flexion-extension et un degré de liberté en rotation correspondant à une rotation du tibia autour de son axe longitudinal. Une telle prothèse doit certainement être considérée comme ayant apporté des solutions pratiques, convenables et acceptables.

Un brevet US 5 011 496 a proposé une prothèse totale du genou qui comprend un élément prothétique fémoral en forme de « U », délimitant un logement d'emboîtement de la partie épiphysaire réséquée d'un fémur et comprenant une partie antérieure définissant une trochlée, par sa face avant, et une partie distalo-postérieure, délimitant deux condyles entre lesquels ladite partie forme un massif présentant, dans sa face extérieure, en considération du logement, un mentonnet se raccordant à la trochlée et bordant un alvéole à partir duquel une portée, à section droite rectangulaire, se développe jusqu'à la partie extrême du massif.

Cette prothèse comprend, en outre, un élément prothétique tibial, constitué d'une embase d'adaptation sur la partie épiphysaire réséquée d'un tibia. L'embase offre, alors, un plateau d'appui plan à un insert qui, d'une part, est monté sur l'embase par l'intermédiaire d'un doigt engagé dans un alésage de l'embase, de manière à être mobile en rotation selon un axe perpendiculaire au plateau d'appui de l'embase et qui, d'autre part, présente, en vis-à-vis de l'élément prothétique fémoral, deux glènes de coopération avec les condyles. L'insert comporte, entre les glènes, une éminence d'orientation sagittale, délimitant, en considération du bord frontal dudit insert, une saillie de stabilisation antéro-postérieure, engagée dans l'alvéole de l'élément fémoral, en position d'extension de la prothèse. La saillie de stabilisation est, par ailleurs, raccordée à une rampe de glissement se développant jusqu'au bord arrière de l'insert et étant destinée à coopérer avec la portée de l'élément fémoral.

La rampe présente une section droite de forme exactement complémentaire de celle de la portée et empêche tout mouvement en dehors du plan sagittal, entre l'insert et l'élément fémoral, de sorte que le seul mouvement possible, en dehors du plan sagittal, reste la rotation entre l'insert et le plateau de l'embase.

Cependant, il est apparu qu'une prothèse, telle qu'évoquée ci-dessus, ne permet pas de maintenir la stabilisation antéro-postérieure, ni d'assurer le glissement ou déplacement relatif arrière du point de contact de l'épiphyse basse du fémur par rapport à l'épiphyse haute du tibia lors de la flexion, déplacement dénommé "roll back", tout en offrant une possibilité de rotation partielle, comme celle autorisée par l'articulation naturelle du genou.

Or, il est apparu, de plus en plus clairement, que ces exigences correspondaient à des besoins anatomiques qu'il importait de maintenir dans le cas de restauration prothétique articulaire, au moins dans le but de réduire la fatigue ligamentaire qu'une prothèse peut induire.

Il est donc apparu le besoin d'un nouveau type de prothèse totale du genou susceptible d'offrir une cinématique qui reproduise au mieux la cinématique anatomique, afin d'offrir, en plus, un grand confort au patient.

Afin d'atteindre cet objectif, l'invention concerne :
- un élément prothétique fémoral en forme de "U" délimitant un logement d'emboîtement de la partie épiphysaire réséquée d'un fémur et comprenant une partie antérieure définissant une trochlée par sa face avant et une partie distalo-postérieure délimitant deux condyles entre lesquels ladite partie forme un massif présentant, dans sa face extérieure en considération du logement, un mentonnet se raccordant à la trochlée et bordant un alvéole à partir duquel une portée se développe jusqu'à la partie extrême du massif,
- et un élément prothétique tibial comprenant, une embase d'adaptation sur la partie épiphysaire réséquée d'un tibia offrant un plateau d'appui plan à un insert qui, d'une part, est monté sur l'embase, par l'intermédiaire d'un doigt engagé dans un alésage de l'embase, de manière à être mobile en rotation selon un axe perpendiculaire au plateau d'appui de l'embase et qui , d'autre part, présente en vis-à-vis de l'élément prothétique fémoral, deux glènes (de coopération avec les condyles, ledit élément tibial comportant, entre les glènes, une éminence d'orientation sagittale délimitant, en considération du bord frontal dudit insert, une saillie de stabilisation antéro-postérieure, engagée dans l'alvéole de l'élément fémoral en position d'extension de la prothèse et raccordée à une rampe de glissement se développant jusqu'au bord arrière de l'insert.

Selon l'invention, la prothèse de genou est caractérisée en ce que :
■ la portée de l'élément prothétique fémoral possède, d'une part, en section droite transversale par rapport à un plan sagittal, une forme concave, sans arête vive, et, d'autre part, en section droite selon le plan sagittal, une forme convexe,
■ et la rampe de glissement de l'insert tibial est destinée à coopérer avec la portée de l'élément fémoral et possède, d'une part, en section droite transversale par rapport au plan sagittal, une forme convexe, douce sans arête vive, et, d'autre part, en section droite selon le plan sagittal, une forme en partie au moins concave adaptée pour induire, en coopération avec la portée de l'élément fémoral, un mouvement de recul vers l'arrière, dit de « roll back » du fémur lors de la flexion de la prothèse.

Le genou selon l'invention permet donc, d'une part, en raison de la liaison pivot entre l'embase et le plateau, une plus grande facilité de mise en place et de réglage du genou appareillé et, d'autre part, en raison de la possibilité de « roll back » une moindre sollicitation des ligaments et un plus grand confort pour le patient.

Selon une caractéristique de l'invention et afin de favoriser le mouvement de « roll back », la rampe de l'insert tibial présente, en section droite selon le plan sagittal, dans une partie antérieure, un rayon de courbure inférieur au rayon de courbure d'une se partie postérieure.

Selon une autre caractéristique de l'invention et afin de conférer au mouvement de « roll back » une meilleure cinématique, la rampe de l'insert tibial présente, en section droite selon le plan sagittal, une partie antérieure incurvée et concave qui se raccorde à une partie postérieure rectiligne.

Selon une encore autre caractéristique de l'invention, la rampe de l'insert tibial présente, en section droite selon le plan sagittal et en tous points de cette section, un rayon de courbure supérieur ou égal à un rayon de courbure minimum qui est compris entre 10 mm et 30 mm et, de préférence, compris entre 13 mm et 25 mm. Ces caractéristiques dimensionnelles de la rampe de l'insert tibial permettent alors d'offrir des conditions de « roll back » optimale

Selon une caractéristique de l'invention, afin de reproduire au mieux le mouvement naturel du genou, la rampe de l'insert tibial présente, en section droite transversale au plan sagittal, un rayon de courbure au sommet compris entre 10 mm et 30 mm et, de préférence, compris entre 14 mm et 24 mm.

Selon encore une autre caractéristique de l'invention, la portée de l'élément fémoral présente une section droite au plan sagittal, un rayon de courbure au fond compris entre 15 mm et 30 mm et, de préférence, compris entre 17 mm et 27 mm.

Par ailleurs, il est apparu que le meilleur mouvement de la prothèse, afin de reproduire aussi fidèlement que possible la cinématique d'un genou naturel, était obtenu lorsque la portée de l'élément fémoral présente en section droite selon le plan sagittal, une région antérieure et une région postérieure qui possèdent un rayon de courbure inférieur au rayon de courbure d'une région médiane séparant les régions antérieure et postérieure. Bien entendu le rayon de courbure de la région antérieure n'est pas nécessairement égal au rayon de courbure de la région postérieure. De même, les régions antérieure, médiane et postérieure ne présentent pas nécessairement un rayon de courbure constant. Cependant, afin d'assurer une fluidité de la flexion de la prothèse les régions antérieure, médiane et postérieure seront de préférence tangentes les unes aux autres au niveau de leurs zones de raccordement.

Selon une caractéristique de l'invention, la région antérieure possède, en section droite selon le plan sagittal, un rayon de courbure supérieur au rayon de courbure de la région postérieure.

Selon encore une autre caractéristique de l'invention permettant d'obtenir une cinématique de flexion optimale, la portée de l'élément fémoral possède, en section droite selon le plan sagittal :
■ dans une région antérieure, un rayon de courbure compris entre 5 mm et 20 mm et, de préférence, compris entre 8 mm et 14 mm,
■ dans une région médiane, un rayon de courbure compris entre 10 mm et 15 mm et, de préférence, compris entre, 12 mm et 20 mm,
■ et dans une région postérieure, un rayon de courbure compris entre 5 mm et 20 mm et, de préférence, compris entre 10 mm et 18 mm.

Bien entendu, les caractéristiques dimensionnelles ne doivent pas être considérées comme strictement nécessaires à l'obtention d'une prothèse de genou selon l'invention.

Selon une autre caractéristique de l'invention et dans une forme préférée mais non strictement nécessaire de réalisation de la prothèse de genou, les axes du doigt de l'insert et de l'alésage de l'embase sont situés dans la moitié antérieure de l'élément tibial, permettant ainsi de positionner, à l'avant, le centre de rotation relatif entre l'insert et son embase, de manière à annuler, autant que faire se peut, la transmission du couple de rotation de l'implant fémoral à l'insert tibial et de privilégier le déplacement linéaire de la partie postérieure de la glène externe par rapport au déplacement linéaire de la partie antérieure de la glène interne.

Selon une autre caractéristique de l'invention, le plateau d'appui de l'embase tibiale présente une surface supérieure à celle du plan d'appui correspondant de l'insert tibial, de manière à permettre une rotation de quelques degrés de l'insert par rapport à la base, sans débordement de l'insert par rapport à l'embase, au moins au niveau de la partie antérieure de l'embase. Cette caractéristique avantageuse de l'invention permet ainsi de limiter, voire d'empêcher tout contact entre l'insert tibial et les ligaments de l'articulation du genou qui auront été conservés.

Dans une forme de réalisation, préférée mais non strictement nécessaire, la partie de l'embase tibiale est dimensionnée de manière à autoriser une rotation d'amplitude inférieure à 15°, sans débordement de l'insert par rapport à l'embase, au moins dans la partie antérieure de l'élément tibial.

Selon une caractéristique de l'invention, afin d'assurer un parfait guidage de la rotation relative entre l'insert et l'embase tibiale et une bonne transmission des efforts subis par l'insert à l'embase, le doigt de l'insert possède une base tronconique se prolongeant, par une extrémité cylindrique, l'alésage de l'embase tibiale présentant alors, au jeu près, une forme complémentaire de celle du doigt de l'insert tibial.

Selon une autre caractéristique de l'invention, toujours en vue de réduire les risques de conflit entre l'insert tibial et les ligaments éventuellement conservés lors de la mise en place de la prothèse de genou selon l'invention, l'insert tibial présente, à l'avant de la saillie de stabilisation et au niveau de l'éminence une cuvette pour le passage au moins du tendon rotulien.

Selon encore une autre caractéristique de l'invention, l'élément prothétique fémoral présente, en avant du mentonnet et dans le prolongement, un sillon trochléen, offrant une surface de guidage pour une rotule naturelle ou prothétique. De manière préférée, l'axe du sillon trochléen forme, avec le plan sagittal, un angle compris entre 6° et 9° et, de préférence, compris entre 7° et 8°.

Selon encore une autre caractéristique de l'invention, le sillon trochléen possède une profondeur croissante à partir de l'extrémité de la trochlée vers le mentonnet et, de préférence mais non exclusivement, comprise entre 0,5 mm et 6 mm.

Diverses autres caractéristiques de l'invention ressortent de la description ci-dessous, effectuée en relation avec les figures annexées qui illustrent une forme préférée, mais non limitative, de réalisation d'une prothèse de genou selon l'invention.

La **fig. 1** est une coupe-élévation prise sensiblement selon le plan sagittal de la prothèse considérée dans un état d'implantation, entre le fémur et le tibia d'un sujet en position d'extension du genou.

La **fig. 2** est une coupe, à plus grande échelle, dans le plan sagittal, montrant une caractéristique constructive d'un exemple de réalisation de l'élément prothétique fémoral.

La **fig. 3** est une élévation de l'élément fémoral d'une prothèse gauche, prise selon la ligne **III-III** de la **fig. 2.**

La **fig. 4** est une coupe dans le plan sagittal, analogue à la **fig. 2,** mais concernant l'insert de l'élément prothétique tibial complémentaire à l'élément fémoral de la **fig. 3**.

La **fig. 5** est une coupe transversale, prise selon la ligne **V-V** de la **fig. 4.**

La **fig. 6** est une coupe, analogue à la **fig. 2**, montrant l'embase de l'élément prothétique tibial.

La **fig. 7** est une élévation selon la ligne **VII-VII** de la **fig. 6.**

La **fig. 8** est une coupe, analogue à la **fig. 2,** accompagnée de sections droites transversales montrant une caractéristique constructive d'un sillon trochléen.

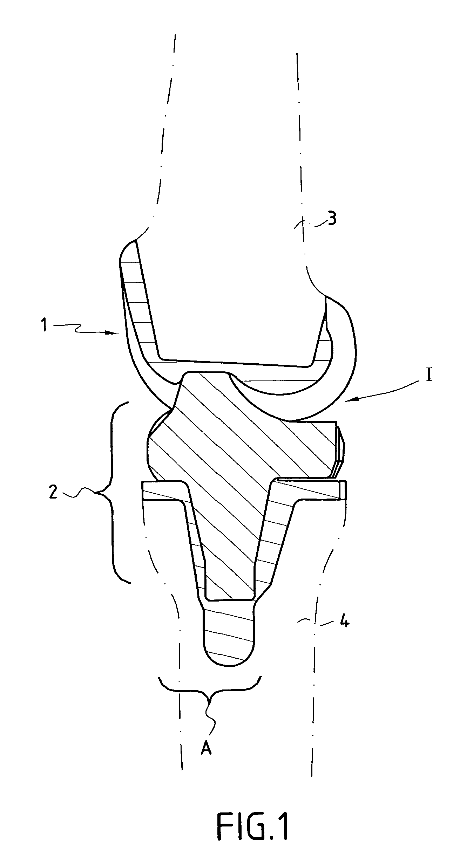

La **fig. 1** montre la prothèse selon l'invention, constituée par un élément prothétique fémoral **1** et par un élément prothétique tibial **2**, destinés à être adaptés, respectivement, après résection osseuse, sur l'épiphyse basse fémorale **3** et sur l'épiphyse haute tibiale **4**.

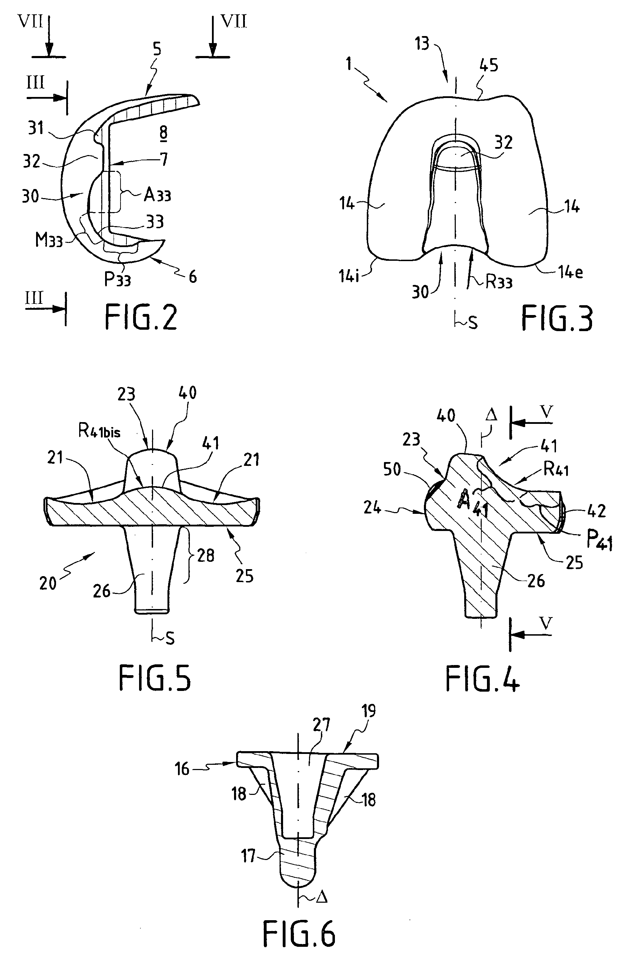

L'élément fémoral **1**, qui peut être réalisé en tout matériau adapté connu de l'homme du métier, présente, selon un plan sagittal et comme le montre la **fig. 2**, une forme sensiblement en "U" comprenant une partie ou branche **5**, dite avant ou antérieure, une partie ou branche **6**, dite arrière ou postérieure, généralement de plus faible longueur que la branche **5**, et une âme **7** de liaison entre ces branches.

La surface interne, délimitée par l'élément fémoral, définit, en quelque sorte, un logement polygonal **8** destiné à emboîter l'épiphyse **3** soumise, préalablement, à une résection complémentaire.

L'élément fémoral est conformé pour présenter, par la surface extérieure de sa grande branche **5**, une surface rotulienne **13**, telle qu'illustrée à la **fig. 3**, délimitant, de façon connue, une trochlée apte à coopérer avec la protubérance naturelle ou avec un bouton artificiel présenté ou porté par la rotule, non représentée.

L'élément fémoral **1** forme, par sa surface extérieure correspondant à l'âme **7** et à la branche **6**, deux condyles **14** qui définissent une partie distale et une partie postérieure.

Il est possible de distinguer, à la **fig. 3** représentant une prothèse tibiale gauche, le condyle interne **14i** et le condyle externe **14e.**

Dans une forme préférée de réalisation, les condyles interne **14i** et externe **14e** présentent une forme anatomique et sont donc asymétriques.

Ainsi, le condyle interne **14i** est petit et rond, tandis que le condyle externe **14e** est plus long et présente une forme plus carrée. Enfin, les condyles interne **14i** et externe **14e** divergent vers l'arrière.

Les condyles **14** sont destinés à coopérer, par leurs parties distale et postérieure, avec l'élément prothétique tibial **2** dont les éléments constitutifs sont représentés plus en détail aux **fig. 4** à **6.** Ainsi, selon l'invention, l'élément tibial **2** comprend une embase **16,** représentée à la **fig. 6,** dont la face inférieure est pourvue d'au moins une queue **17,** éventuellement renforcée par des goussets **18**. La queue **17** est destinée à être implantée dans l'épiphyse tibiale **4**, avec ou sans présence d'un ciment de liaison. Comme cela est connu, la fixation de l'embase **16** peut aussi faire intervenir la présence de vis, non représentées.

L'embase tibiale 16 offre un plateau d'appui **19**, de préférence plan, pour un insert **20**, de préférence réalisé en une matière plastique appropriée, telle qu'en polyéthylène. Comme le montre la **fig. 5**, l'insert **20** est conformé pour offrir, sur son dessus, deux glènes **21** destinées à coopérer avec les surfaces extérieures des condyles **14** et une épine **23** formée, dans la partie sagittale, à partir du bord antérieur **24** et entre les glènes **21**.

L'insert **20** est monté sur l'embase **16**, de manière à être mobile en rotation par rapport à cette dernière. A cet effet, l'insert **20** possède une face inférieure plane **25** d'appui sur le plateau **19.** L'insert présente, en outre, à partir de cette face plane **25,** un doigt **26** destiné à venir s'engager dans un alésage complémentaire **27**, aménagé dans l'embase **16,** de manière à définir une liaison pivot d'axe Δ sensiblement contenu dans le plan sagittal **S**.

Selon l'exemple illustré, le doigt **26** présente, à partir du plan **25**, une base **28** de forme tronconique, se prolongeant par une extrémité **29** de forme cylindrique. Bien entendu, l'alésage **27** possède une forme sensiblement analogue. Cette conformation de l'alésage **27** et du doigt **26** permet, de manière très avantageuse, une très bonne transmission des efforts subis par l'insert **20** à l'embase **16**. Toutefois, une autre conformation à géométrie de révolution, telle que cylindrique seulement, pourrait également être adoptée pour le doigt **26** et l'alésage **27**.

Par ailleurs, il doit être remarqué, selon l'exemple illustré, que l'axe Δ, perpendiculaire au plateau **19** du doigt **26** et de l'alésage **27,** se situe dans la moitié antérieure A de l'élément prothétique fémoral **2**.

Conformément à l'invention, l'élément prothétique fémoral **1** est réalisé pour comporter, dans sa partie inter-condylienne, un sillon **30** qui est conformé pour que sa surface extérieure, en considération du logement **8**, soit située en retrait par rapport à celle des condyles 14. Cette surface en retrait est aménagée pour former, dans la partie de liaison avec la branche **5,** un mentonnet **31** qui délimite une sorte d'alvéole **32,** à la suite duquel se développe une portée **33** aboutissant à la partie extrême arrière de la branche **6**.

Comme le montre plus particulièrement la **fig. 2**, la portée **33** présente, en section droite selon le plan sagittal **S**, une forme convexe, tandis qu'en section droite, prise perpendiculairement au plan sagittal **S**, la portée **33** présente une forme concave qui apparaît plus particulièrement à la **fig. 3.** Il doit être remarqué que la forme convexe de la portée **33** en coupe selon le plan sagittal est réalisée de manière à assurer une transition, douce et sans arête, avec les condyles **14e** et **14i**. De manière préférée mais non strictement nécessaire, la concavité transversale de la portée **33** est aménagée de manière à présenter un rayon de courbure **R**_{**33**}**,** de préférence mais non nécessairement, compris entre 15 mm et 30 mm et, de façon plus particulièrement préférée, entre 17 mm et 27 mm.

Par ailleurs, la portée **33** de l'élément fémoral est aménagée de façon à connaître un rayon de courbure, dans le plan sagittal, différent dans trois régions distinctes de la portée, à savoir une région antérieure à **A**_{**33**}**,** une région médiane **M**_{**33**} et une région postérieure **P**_{**33**}**.** Ainsi, de manière préférée, le rayon de courbure de la portée **33** dans la région antérieure est choisi de manière à être compris entre 5 mm et 20 mm et, de manière plus particulièrement préférée, entre 8 mm et 14 mm. De la même manière, le rayon de courbure dans la région médiane de la portée **33** est choisi pour être compris, de préférence, compris entre 10 mm et 25 mm et, de manière plus particulièrement préférée, compris entre 12 mm et 20 mm.

Enfin, dans la région postérieure, le rayon de courbure de la portée **33** sera choisi pour être de préférence compris entre 5 mm et 20 mm et, de manière plus particulièrement préférée, compris entre 10 mm et 18 mm.

Les valeurs des rayons de courbure dans les régions antérieure, médiane et postérieure de la portée **33** seront alors choisies en fonction de la taille de la prothèse et de la nature du mouvement de roll back recherché.

De façon complémentaire, l'insert **20** de l'élément tibial comprend, le long de l'épine **23,** une saillie **40**, de forme complémentaire à l'alvéole **32,** formée en retrait du bord antérieur **24** et raccordée à une rampe **41** qui se développe sur l'axe sagittal en direction du bord postérieur **42** de l'insert **20**. La rampe **41**, dite de glissement, affecte, dans le plan sagittal, une forme générale concave.

Par ailleurs, comme le montre plus particulièrement la **fig. 4,** la rampe **41** est conformée de manière à présenter, en section droite selon le plan sagittal et dans sa partie antérieure **A**_{**41**}**,** une forme sensiblement circulaire, présentant un rayon de courbure **R**_{**41**}**,** choisi pour être compris, de manière préférée, entre 10 mm et 30 mm et, de manière plus particulièrement préférée, entre 13 mm et 25 mm. De plus, la rampe **41** se prolonge, dans sa partie postérieure **P**_{**41**}**,** par une portion qui pourrait être considérée, vue dans le plan sagittal, comme étant rectiligne. Ainsi, il peut être considéré que la rampe de l'insert tibial présente, en section droite selon le plan sagittal et tous points de cette section, un rayon de courbure supérieur au rayon de courbure **R**_{**41**} qui pourrait être qualifié de rayon de courbure minimum de l'insert dans le plan sagittal **S.**

Conformément à une caractéristique essentielle de l'invention, la rampe **41** présente, en section droite transversale au plan sagittal, comme le montre la **fig. 5,** une forme convexe, douce et sans arête, qui sera choisie de manière à présenter, de façon préférée mais non strictement nécessaire, un rayon de courbure au sommet **R**_{**41bis**} compris entre 10 mm et 30 mm et, de manière plus particulièrement préférée, compris entre 14 mm et 24 mm.

La coopération des surfaces en vis-à-vis des éléments prothétiques **1** et **2**, savoir l'alvéole **32**, la rampe **33**, la saillie **40** et la rampe **41**, conduit à une situation relationnelle dans un état d'extension, tel qu'illustré dans la **fig. 1**, dans laquelle la saillie **40** est totalement engagée dans l'alvéole **32** et se trouve en coopération de surface avec le mentonnet **31** et la portion de surface **34**, alors que, simultanément, les condyles **14** coopèrent avec les glènes **21**

Dans cet état, la portée **33** et la rampe **41** définissent entre elles et à partir de l'alvéole **32,** un intervalle **I** dont la section croît depuis cet alvéole en direction de la partie ou branche postérieure **6**.

Comme cela ressort de la **fig. 1**, dans une telle situation relationnelle, la coopération entre la saillie **40** et l'alvéole **32** produit une stabilisation antéro-postérieure efficace, sans risque de glissement dans le plan sagittal.

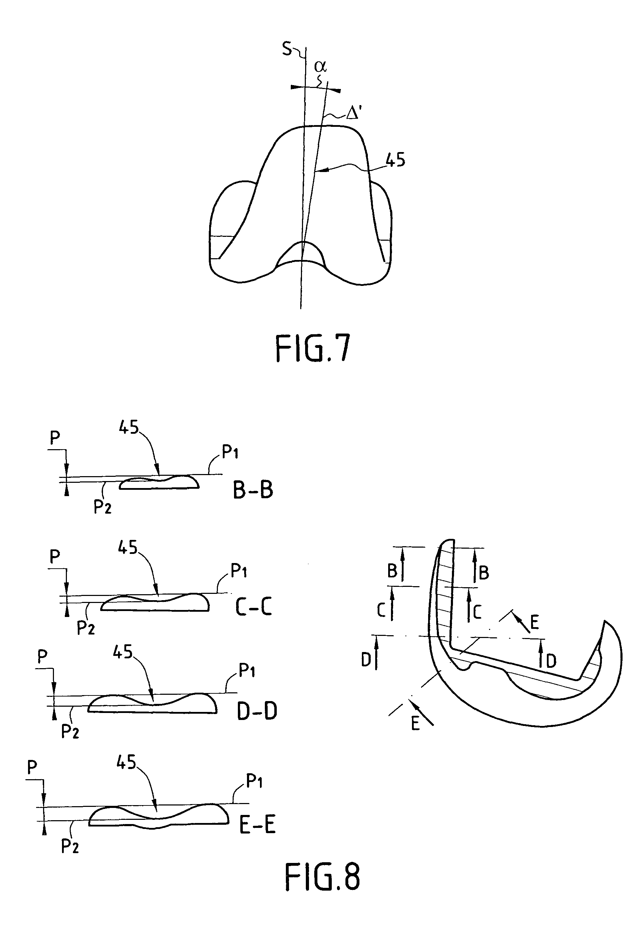

Par ailleurs, la concavité de la portée **33** permet de réduire au maximum les risques d'interférence avec la rotule, naturelle ou prothétique, du genou appareillé. Cette caractéristique avantageuse de l'invention est complétée par la mise en oeuvre d'un sillon trochléen **45** destiné à assurer le guidage de la rotule, naturelle ou prothétique. Comme le montre la **fig. 7,** l'axe Δ' du sillon trochléen **45** forme un angle α par rapport au plan sagittal **S.** De manière préférée, l'angle α sera compris entre 6° et 9° et, de façon plus particulièrement préférée, entre 7° et 8°, α = 7,5° pouvant constituer un compromis acceptable.

Par ailleurs, comme le montre la **fig. 8**, le sillon trochléen est réalisé de manière à présenter une profondeur croissante depuis sont extrémité jusqu'à l'alvéole **32**, comme cela ressort plus particulièrement des sections **B-B, C-C, D-D** et **E-E.** Cette profondeur **P,** mesurée entre le plan **P**_{**1**} tangent aux sommets de la trochlée adjacents au sillon **45** et le plan **P**_{**2**} parallèle à **P**_{**1**} passant par le fond du sillon **45,** est alors, de préférence, comprise entre 0,5 mm et 6 mm. De façon préférée, la profondeur **P** en début de sillon **45** (section **B-B)** est comprise entre 0,5 mm et 2,5 mm, tandis que la profondeur **P,** mesurée en fin de sillon **45** au voisinage de l'alvéole **32** (section **E-E**) est comprise entre 3,5 mm et 5,7 mm.

Il doit être remarqué que, selon l'exemple illustré et comme le montre plus particulièrement la **fig. 1**, l'insert **20** est conformé de manière à présenter, en projection sur le plan du plateau **19**, une surface inférieure à celle dudit plateau, de manière que, lors d'une rotation relative de l'insert par rapport à l'embase, la partie antérieure de l'insert ne déborde pas par rapport au plateau **19** ou à l'embase, réduisant ainsi les risques d'interférences de l'insert avec les tissus ou les ligaments de l'articulation appareillée.

Par ailleurs, toujours dans un souci d'une meilleure adaptation au genou appareillé, selon l'exemple illustré, l'insert présente, dans sa partie antérieure et au niveau de l'éminence **23**, à l'avant de la saillie de stabilisation **40**, une cuvette **50** pour le passage des tissus du genou appareillé et, plus particulièrement, de la rotule et de son tendon.

Bien entendu, diverses modifications peuvent être apportées à la prothèse de genou, telle que décrite ci-dessus, sans sortir du cadre de la présente invention.

## Revendications

1. Prothèse de genou comprenant :
• un élément prothétique fémoral **(1)** en forme de "U" délimitant un logement d'emboîtement **(8)** de la partie épiphysaire réséquée **(3)** d'un fémur et comprenant une partie antérieure **(5)** définissant une trochlée **(13)** par sa face avant et une partie distalo-postérieure **(6)** délimitant deux condyles **(14i, 14e)** entre lesquels ladite partie forme un massif présentant, dans sa face extérieure en considération du logement, un mentonnet **(31)** se raccordant à la trochlée et bordant un alvéole **(32)** à partir duquel une portée **(33)** se développe jusqu'à la partie extrême du massif,
• et un élément prothétique tibial **(2)** comprenant, une embase **(16)** d'adaptation sur la partie épiphysaire réséquée **(4)** d'un tibia offrant un plateau d'appui plan **(19)** à un insert **(20)** qui, d'une part, est monté sur l'embase, par l'intermédiaire d'un doigt **(26)** engagé dans un alésage **(27)** de l'embase, de manière à être mobile en rotation selon un axe (Δ) perpendiculaire au plateau d'appui de l'embase et qui, d'autre part, présente, en vis-à-vis de l'élément prothétique fémoral, deux glènes **(21)** de coopération avec les condyles **(14)**, ledit élément tibial comportant, entre les glènes, une éminence **(23)** d'orientation sagittale délimitant, en considération du bord frontal dudit insert, une saillie **(40)** de stabilisation antéro-postérieure, engagée dans l'alvéole **(32)** de l'élément fémoral **(1)** en position d'extension de la prothèse et raccordée à une rampe **(41)** de glissement pour la portée **(33)** se développant jusqu'au bord arrière de l'insert,
**caractérisée en ce que** :
■ la portée **(33)** de l'élément prothétique fémoral **(1)** possède, d'une part, en section droite transversale par rapport à un plan sagittal, une forme concave, douce sans arête vive, et, d'autre part, en section droite selon le plan sagittal, une forme convexe,
■ et la rampe de glissement **(41)** de l'insert tibial **(2)** est destinée à coopérer avec la portée **(33)** de l'élément fémoral **(1)** et possède, d'une part, en section droite transversale par rapport au plan sagittal, une forme convexe, douce sans arête vive, et, d'autre part, en section droite selon le plan sagittal, une forme en partie au moins concave adaptée pour induire, en coopération avec la portée **(33)** de l'élément fémoral **(1)**, un mouvement de recul vers l'arrière, dit de « roll back » du fémur lors de la flexion de la prothèse.

2. Prothèse du genou selon la revendication 1, **caractérisée en ce que** la rampe **(41)** de l'insert tibial **(2)** présente, en section droite selon le plan sagittal **(S),** dans une partie antérieure **(A**_{**41**}**),** un rayon de courbure inférieur au rayon de courbure de sa partie postérieure **(P**_{**41**}**).**

3. Prothèse du genou selon la revendication 1 ou 2, **caractérisée en ce que** la rampe **(41)** de l'insert tibial **(2)** présente, en section droite selon le plan sagittal (S), une partie antérieure incurvée et concave qui se raccorde à une partie postérieure rectiligne.

4. Prothèse du genou selon la revendication 3 **caractérisée en ce que** la rampe **(41)** de l'insert tibial **(2)** présente, en section droite selon le plan sagittal et dans sa partie antérieure **(A**_{**41**}**),** une forme sensiblement circulaire.

5. Prothèse du genou selon l'une des revendications 1 à 4, **caractérisée en ce que** la rampe **(41)** de l'insert tibial **(2)** présente, en section droite selon le plan sagittal **(S)** et en tous points de cette section, un rayon de courbure **(R**_{**41**}**)** supérieur ou égal à un rayon de courbure minimum qui est compris entre 10 mm et 30 mm et, de préférence, compris entre 13 mm et 25 mm.

6. Prothèse du genou selon l'une des revendications 1 à 5, **caractérisée en ce que** la rampe **(41)** de l'insert tibial présente, en section droite transversale au plan sagittal **(S),** un rayon de courbure au sommet **(R**_{**41**}**)** compris entre 10 mm et 30 mm et, de préférence, compris entre 14 mm et 24 mm.

7. Prothèse du genou selon l'une des revendications 1 à 6, **caractérisée en ce que** la portée **(33)** de l'élément fémoral **(1)** présente, en section droite selon le plan sagittal **(S),** une région antérieure **(A**_{**33**}**)** et une région postérieure **(P**_{**33**}**)** qui possèdent un rayon de courbure inférieur au rayon de courbure d'une région médiane **(M**_{**33**}**)** séparant les régions antérieure **(A**_{**33**}**)** et postérieure **(P**_{**33**}**).**

8. Prothèse du genou selon l'une des revendications 1 à 7, **caractérisée en ce que** la région antérieure **(A**_{**33**}**)** possède, en section droite selon le plan sagittal **(S),** un rayon de courbure supérieur au rayon de courbure de la région postérieure **(P**_{**33**}**).**

9. Prothèse du genou selon l'une des revendications 1 à 8, **caractérisée en ce que** la portée **(33)** de l'élément fémoral **(1)** présente, en section droite selon le plan sagittal :
■ dans une région antérieure **(A**_{**33**}**),** un rayon de courbure compris entre 5 mm et 20 mm et, de préférence, compris entre 8 mm et 14 mm,
■ dans une région médiane **(M**_{**33**}**),** un rayon de courbure compris entre 10 mm et 25 mm et, de préférence, compris entre 12 mm et 20 mm,
■ et dans une région postérieure **(P**_{**33**}**),** un rayon de courbure compris entre 5 mm et 20 mm et, de préférence, compris entre 10 et 18 mm.

10. Prothèse du genou selon l'une des revendications 1 à 9, **caractérisée en ce que** la portée **(33)** de l'élément fémoral **(1)** présente, en section droite transversale au plan sagittal **(S),** un rayon de courbure **(R**_{**33**}**)** au fond compris entre 15 mm et 30 mm et, de préférence, compris entre 17 mm et 27 mm.

11. Prothèse de genou selon l'une des revendications 1 à 10, **caractérisée en ce que** l'axe (Δ) du doigt **(21)** de l'insert **(20)** et de l'alésage **(27)** de l'embase **(16)** sont situés dans la moitié antérieure **(A)** de l'élément tibial **(2).**

12. Prothèse de genou selon l'une des revendications 1 à 11, **caractérisée en ce que** le plateau **(19)** d'appui de l'embase tibiale **(16)** présente une surface supérieure à celle du plan d'appui **(25)** correspondant de l'insert tibial **(20)**, de manière à permettre une rotation de quelques degrés de l'insert par rapport à l'embase, sans débordement de l'insert **(20)** par rapport à l'embase **(16)**, au moins au niveau de la partie antérieure de l'élément tibial.

13. Prothèse de genou selon la revendication 12, **caractérisée en ce que** le plateau d'appui **(19)** de l'embase tibiale **(16)** est dimensionné de manière à autoriser une rotation d'amplitude inférieure à 15°, sans débordement de l'insert par rapport à l'embase, au moins dans la partie antérieure de l'élément tibial.

14. Prothèse de genou selon l'une des revendications 1 à 13, **caractérisée en ce que** le doigt de rotation **(26)** de l'insert **(20)** possède une base tronconique **(28)** se prolongeant par une extrémité cylindrique, l'alésage **(27)** de l'embase tibiale **(16)** présentant, au jeu près, une forme complémentaire de celle du doigt de l'insert tibial.

15. Prothèse de genou selon l'une des revendications 1 à 14, **caractérisée en ce que** l'insert tibial **(20)** présente, à l'avant de la saillie de stabilisation et au niveau de l'éminence, une cuvette **(50)** pour le passage d'au moins un ligament de maintien rotulien.

16. Prothèse de genou selon l'une des revendications 1 à 15, **caractérisée en ce que** l'élément fémoral comprend un sillon trochléen **(45)** d'axe (Δ') formant, avec le plan sagittal **(S),** un angle compris entre 6° et 9° et, de préférence, compris entre 7° et 8°.

17. Prothèse de genou selon la revendication 16, **caractérisée en ce que** le sillon trochléen **(45)** possède une profondeur **(P),** croissante à partir de l'extrémité de la trochlée et vers le mentonnet **(31)**, comprise entre 0,5 mm et 6 mm.
